(19) Europäisches Patentamt
European Patent Office
Office européen des brevets

(11) Veröffentlichungsnummer: **0 161 464 B1**

# (12) EUROPÄISCHE PATENTSCHRIFT

(45) Veröffentlichungstag der Patentschrift: **24.07.91**

(51) Int. Cl.⁵: **C08F 34/02**, C08F 8/12, C12N 11/08

(21) Anmeldenummer: **85104099.8**

(22) Anmeldetag: **04.04.85**

## (54) Polymerisate auf Basis von Polyvinylencarbonat und/oder Polyhydroxymethylen, Verfahren zur ihrer Herstellung und ihre Anwendung.

(30) Priorität: **13.04.84 DE 3413904**

(43) Veröffentlichungstag der Anmeldung:
**21.11.85 Patentblatt 85/47**

(45) Bekanntmachung des Hinweises auf die Patenterteilung:
**24.07.91 Patentblatt 91/30**

(84) Benannte Vertragsstaaten:
**AT BE CH DE FR GB IT LI LU NL SE**

(56) Entgegenhaltungen:
**EP-A- 0 110 281**
**DE-A- 2 014 242**
**DE-A- 2 556 759**
**GB-A- 1 009 004**

(73) Patentinhaber: **HOECHST AKTIENGESELLSCHAFT**
**Postfach 80 03 20**
**W-6230 Frankfurt am Main 80(DE)**

(72) Erfinder: **Mauz, Otto, Dr.**
**Heidestrasse 21**
**W-6237 Liederbach(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die Herstellung von Polyvinylencarbonat mit hohem Molekulargewicht durch radikalisch initiierte Polymerisation des Monomeren in Substanz oder in Lösung ist bekannt (vgl. J. Polymer Sci. 58 (1962), Seiten 533 ff.) Gleiches gilt auch für die Copolymerisation von Vinylencarbonat mit verschiedenen Vinylverbindungen (vgl. US-Patentschriften 2.722.525; 2.847.398; 2.847.401; 2.847.402; 2.934.514; 2.945.836 und 2.957.847). Vinylencarbonatpolymere haben Anwendung gefunden als Plastikmaterial, als Bindemittel und als Imprägniermittel (vgl. US-Patentschriften 2.794.013 und 2.930.779).

Es ist auch bekannt, daß durch saure oder alkalische Hydrolyse Polyvinylencarbonat in Polyhydroxymethylen überführt werden kann.

Aus der DE-Offenlegungsschrift 1 494 576 kennt man die Verarbeitung von Polyvinylencarbonat und von dessen Verseifungsprodukt Polyhydroxymethylen zu Filmen, Fasern und Folien.

Aus der DE-Offenlegungsschrift 2 556 759 ist ein Verfahren zur Herstellung von Polymeren und Copolymeren des Vinylencarbonats bekannt, bei welchem die Polymerisation in Gegenwart eines organischen Dispersionsmittels durchgeführt wird und nichtionogene, grenzflächenaktive organische Verbindungen als Dispersionsstabilisatoren zugesetzt werden. Als Dispersionsstabilisatoren werden u.a. auch oxäthylierte gesättigte Fettalkohole genannt. Bei der Durchführung dieses Verfahrens tritt aber bei fortschreitender Polymerisation eine Verklumpung der Polymerteilchen ein. Ein solches Produkt eignet sich wenig zu einer Weiterverarbeitung.

Aufgabe der vorliegenden Erfindung war es nun, ein einfach herstellbares, modifiziertes Polyvinylencarbonat bzw. ein modifiziertes Polyhydroxymethylen mit anpassungsfähiger Hydrophilie zur Verfügung zu stellen, wobei die Einstellung der Hydrophilie im wesentlichen nicht durch Einbau von Comonomeren oder durch Modifizierung der Cyclocarbonatgruppen erfolgen soll.

Zur Lösung dieser Aufgabe schlägt die Erfindung ein Polymerisat vor, das im wesentlichen besteht aus den Einheiten

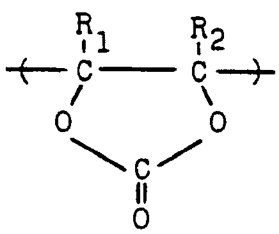 und/oder (I)

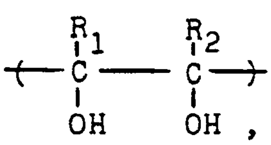 (II)

worin $R_1$ und/oder $R_2$ Wasserstoff oder einen einwertigen Kohlenwasserstoffrest mit bis zu 8 Kohlenstoffatomen bedeuten,
und gegebenenfalls kleiner Mengen von weiteren Monomer-Einheiten,
dadurch gekennzeichnet, daß es noch Einheiten kovalent gebunden enthält, die sich ableiten von mindestens einer Verbindung der Formel

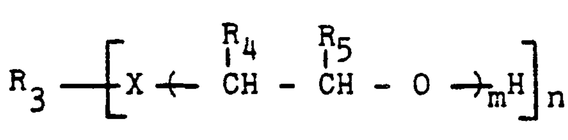 , (III)

worin bedeuten:

$R_3$ = Kohlenwasserstoffrest mit 4 bis 30 Kohlenstoff-Atomen, vorzugsweise einen Alkylrest mit 4

bis 30 Kohlenstoff-Atomen, vorzugsweise 12 bis 20 Kohlenstoff-Atomen oder einen Arylrest mit 6 bis 14 Kohlenstoff-Atomen, vorzugsweise 6 bis 10 Kohlenstoff-Atomen, vorzugsweise einen Phenylrest, wobei dieser Aryl(Phenyl)rest gegebenenfalls mit mindestens einem, vorzugsweise 1 bis 3 Alkylresten mit 4 bis 30 Kohlenstoff-Atomen, vorzugsweise 12 bis 20 Kohlenstoff-Atomen substituiert sein kann;

X = -O-, -NH- und/oder -COO-, vorzugsweise -COO-, und/oder -O-;

$R_4$, $R_5$ = Wasserstoff oder einen einwertigen Kohlenwasserstoffrest, insbesondere einen Alkylrest, mit 1 bis 8 Kohlenstoff-Atomen, mit der Maßgabe, daß mindestens einer der Reste $R_4$, $R_5$ Wasserstoff ist;

m = ganze Zahl von 1 bis 40, vorzugsweise 5 bis 35;

n = ganze Zahl von 1 bis 4, vorzugsweise 1 oder 2.

Weiterhin betrifft die Erfindung ein Verfahren zur Herstellung derartiger Polymerisate durch Polymerisation von Verbindungen der Formel

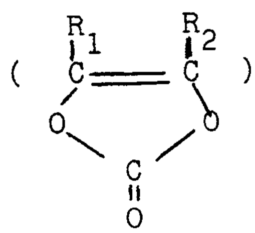

(IV)

worin $R_1$ und $R_2$ die obige Bedeutung haben, und gegebenenfalls kleiner Mengen weiterer damit copolymerisierbarer Monomere, wobei die Polymerisation in einem flüssigen Dispersionsmittel, in Gegenwart eines radikalisch wirksamen Initiators und eines Dispersionsstabilisators durchgeführt wird, und gegebenenfalls Hydrolyse der Cyclocarbonatringe des so erhaltenen Produkts, dadurch gekennzeichnet, daß die Polymerisation in Gegenwart von mindestens einer Verbindung gemäß Formel (III) erfolgt und daß als Dispersionsstabilisator ein Copolymerisat aus Maleinsäureanhydrid und einem Vinylalkyläther und/oder einem Vinylester und/oder einem längerkettigen $\alpha$-Olefin verwendet wird.

Die Erfindung hat schließlich auch noch die Verwendung der so erhaltenen Polymeren, gegebenenfalls nach Umsatz mit Spacern, als Trägermaterialien zur Herstellung von trägergebundenen biologisch aktiven Substanzen, als Adsorptionsmittel in der Affinitätschromatographie bzw. Gelchromatographie oder zur Herstellung von Formkörpern zum Gegenstand.

Das Basispolymere des erfindungsgemäßen Polymerisates besteht im wesentlichen, vorzugsweise zu mindestens 90 Gew.-% aus den obigen Einheiten (I) und/oder (II), wobei $R_1$ und/oder $R_2$ Wasserstoff oder einen einwertigen Kohlenwasserstoffrest mit bis zu 8 Kohlenstoffatomen, vorzugsweise einen Alkylrest mit 1 bis 6 Kohlenstoffatomen und insbesondere einen solchen mit 1 bis 4 Kohlenstoffatomen bedeuten. Beispiele hierfür sind: der Methyl-, Äthyl-, Isopropyl-, 2-Äthylhexyl-, n-Heptyl-, Cyclopentyl-, Phenyl-, Tolyl-, Benzyl- oder Xylylrest.

Bevorzugt stehen die Reste $R_1$ und $R_2$ für Wasserstoff.

Nach einer weiteren bevorzugten Ausgestaltungsform der Erfindung beträgt die Menge an Einheiten (II) mehr als 50 Gew-%, bezogen auf die Gesamtmenge von (I) und (II), d.h. die Cyclocarbonatgruppen von (I) sind mehrheitlich zu Hydroxylgruppen verseift. Insbesondere beträgt die Menge an (II) mindestens 95 Gew-% und besonders bevorzugt 100 Gew-%.

Gegebenenfalls kann das Basispolymere noch geringe Mengen anderer Monomer-Einheiten enthalten, die sich von Monomeren ableiten, die mit Vinylencarbonat bzw. dessen Derivaten copolymerisierbar sind. Diese Monomeren können auch hydrophile oder vernetzende Gruppen aufweisen. Als Beispiele für derartige Monomere seien hier aufgeführt: Vinylpyrrolidon, (Meth)-Acrylsäurealkylester mit jeweils 2 bis 6 C-Atomen in der Alkylgruppe, Hydroxyalkylester der (Meth)Acrylsäure mit 2 bis 6 C-Atomen in der Alkylgruppe, N-Vinyl-N-alkylacetamid ($C_1$-$C_4$-Alkyl), Vinylacetat, Divinyläther von Glykolen wie Äthylenglykoldivinyläther, Butandioldivinyläther, Hexandioldivinyläther, N,N'-Alkylenbis(meth)acrylamide mit bis zu 12 C-Atomen, vorzugsweise bis zu 6 C-Atome enthaltenden geradkettigen oder verzweigten Alkylenresten wie N,N'-Methylenbisacrylamid, N,N'-Äthylenbisacrylamid, N,N'-Hexamethylenbisacrylamid, N,N'-Methylenbismethacrylamid, N,N'-Äthylenbismethacrylamid, N,N'-Hexamethylenbismethacrylamid, N,N'-Äthylidenbisacrylamid, Glyoxalbisacrylamid, (1,2-Bisacrylamidol,2-dihydroxyäthan), Bis-acrylamidoessigsäure, Äthylenglykol-bismethacrylsäure-glykol-bis-methacrylsäureester, Butandiolbismethacrylsäureester, Triallylcyanurat, Trisacryloylperhydrotriazin, Divinylbenzol, Adipinsäuredivinylester, N,N'-Divinylethylenharnstoff, N,N'-Divinylpropylenharnstoff, Ethyliden-bis-3-(N-vinyl-pyrrolidon), N,N'-Divinyldiimidazolyl(2,2') und 1,1'-Bis(3,3'-vinylbenzimidazolid-2-on)-1,4-butan, Vinylacrylat, Allylmethacrylat, Acryl- und Methacrylsäureester mit 5-12 C-Atomen im Alkylrest, (Meth)Acrylnitril, Vinylester mit 4-18 C-Atomen in dem Carbonsaurerest, wie Vinylbutyrat, Vinylstearat, und Vinylester verzweigter Carbonsäuren mit 10 bis 12 C-Atomen; weiterhin Vinylaromaten, wie Styrol oder α-Methylstyrol.

Es können auch mehrere sich von diesen Monomeren ableitende Einheiten im Basispolymeren enthalten sein.

Die Menge dieser Monomer-Einheiten - falls vorhanden - übersteigt im allgemeinen nicht 15 Gew-% und beträgt bevorzugt höchstens 10 Gew-%, jeweils bezogen auf das gesamte Basispolymere. Vernetzende Monomer-Einheiten, beispielsweise in Mengen von 0,2-5 Mol.-%, vorzugsweise 0,5-3 Mol.-% führen zu einer höheren mechanischen Stabilität, was bei Einsatz in der Säulenchromatographie vorteilhaft sein kann. Bevorzugt besteht das Basispolymere jedoch nur aus den Einheiten (I) und/oder (II).

Das Basispolymere enthält als erfindungsgemäße Neuerung solche Einheiten, die sich von mindestens einer Verbindung der Formel (III) ableiten. Diese Einheiten können dabei auch von verschiedenen Verbindungen der Formel (III) stammen, d.h. es können bei der Herstellung der erfindungsgemäßen Polymerisate auch Gemische der verschiedenen Verbindungen gemäß Formel (III) zum Einsatz kommen.

Die Menge dieser Einheiten gemäß (III) beträgt im Regelfall bis zu 30 Gew-%, vorzugsweise 1 bis 20 Gew-%, und insbesondere 7 bis 15 Gew-%, jeweils bezogen auf das Basispolymere.

In der obigen Formel (III) bedeuten die einzelnen Substituenten bevorzugt folgendes:

$R_3$ = verzweigter, vorzugsweise aber unverzweigter Alkylrest mit 4 bis 30 Kohlenstoff-Atomen, vorzugsweise 12 bis 20 Kohlenstoff-Atomen.
Beispielhaft seien genannt: Hexyl-, Heptyl-, Octyl-, Nonyl-, Decyl-, Undecyl-, Dodecyl-, Hexadecyl-, Octadecyl-, Eikosyl-.

X = -O-; -COO-; insbesondere -O-;

$R_4$ und $R_5$ = Wasserstoff oder Methyl, insbesondere Wasserstoff;

m = ganze Zahl von 5 bis 35;

n = 1 oder 2, insbesondere 1.

Bevorzugte Vertreter der Formel (III) sind dementsprechend oxäthylierte, einbasische, aliphatische Carbonsäuren (Alkyl ($C_{4-30}$)), wobei auch Gemische derartiger Säuren (z.B. Fettsäuren) in Frage kommen. Als besonders bevorzugte Vertreter sind hier oxäthylierte einwertige, aliphatische Alkohole (Alkyl ($C_{4-30}$)) zu nennen, die bevorzugt primär sind. Auch hier sind Gemische derartiger Alkohole (z.B. Fettalkohole) brauchbar.

Weniger bevorzugte Vertreter der Formel (III) sind beispielsweise entsprechende oxäthylierte, einwertige, insbesondere primäre Amine (bei X = -NH-), oxäthylierte Hydroxycarbonsäuren (bei X = -O- und -COO-; n = 2), oxäthylierte aromatische Carbonsäuren ($R_3$ = Phenylrest) oder mehrwertige/mehrbasische Alkohole/Carbonsäuren ( n = 1).

Bevorzugte erfindungsgemäße Polymere sind nach den obigen Ausführungen demnach Polyvinylencarbonat und/oder Polyhydroxymethylen, jeweils enthaltend Einheiten von oxäthylierten, einbasischen, aliphatischen Carbonsäuren (Alkyl ($c_{4-30}$)) oder insbesondere oxäthylierten einwertigen, aliphatischen Alkoholen (Alkyl ($C_{4-30}$)) in bevorzugten Mengen von 1 bis 20 Gew-% und insbesondere 7 bis 15 Gew-%.

Das Basispolymere und die Einheiten der Verbindungen gemäß Formel (III) sind erfindungsgemäß durch kovalente Bindungen miteinander verknüpft. Ohne sich festlegen zu wollen, dürfte dabei der überwiegende Teil der im erfindungsgemäßen Polymerisat eingebauten Einheiten gemäß Formel (III) auf das Basispolymere aufgepfropft sein. Daneben ist auch der Einbau als Endgruppen denkbar. Bei größeren Zahlenwerten für m, d.h. bei längeren Polyätherresten kann es auch zu einer Pfropfung von Polyvinylencarbonatketten auf die alkoxylierten Verbindungen gemäß Formel (III) kommen.

Das erfindungsgemäße Verfahren zur Herstellung der beanspruchten Polymeren wird vorzugsweise unter den üblichen bekannten Bedingungen der Perlpolymerisation durchgeführt, wie sie beispielsweise in der DE-Offenlegungsschrift 2 237 316 oder der DE-Offenlegungsschrift 2 556 759 beschrieben sind, jedoch mit der Neuerung, daß spezielle Dispersionsstabilisatoren zum Einsatz kommen.

Bei diesen handelt es sich um vorzugsweise alternierende Copolymere aus Maleinsäureanhydrid und einem Vinylalkyläther, vorzugsweise einem Vinyl-n-alkyläther mit 6 bis 30 C-Atomen, vorzugsweise 10 bis 20 C-Atomen in der Alkylgruppe, oder einem Vinylester mit 6 bis 30 C-Atomen, vorzugsweise 10 bis 20 C-Atomen in der Carbonsäuregruppe oder einem längerkettigen α-Olefin mit 8 bis 30 C-Atomen, vorzugsweise 10 bis 20 C-Atomen. Als Beispiele für derartige Vinylalkyläther, Vinylester bzw. längerkettigen α-Olefinen seien hier genannt: Vinyloctyläther, Vinyldecyläther, Vinyldodecyläther, Vinylstearyläther, Vinylmyricyläther, Ethylhexansäure-Vinylester, Isononansäure-Vinylester, Versaticsäure-Vinylester, Laurinsäure-Vinylester,

Stearinsäure-Vinylester und Vinylester verzweigter Carbonsäuren mit 10 bis 12 C-Atomen; Octen-(1), Decen-(1), Tetradecen-(1), Octadecen-(1), Tricosen-(1).

Diese Dispersionsstabilisatoren, die auch in Mischung eingesetzt werden können, sind bereits in Mengen von 0,001 Gew-%, bezogen auf die Gesamtmenge an Monomeren, wirksam. Zumeist werden Mengen von 0,005 bis 10 Gew-%, vorzugsweise 0,01 - 5 Gew-%, bezogen auf Gesamtmenge an Monomeren, verwendet.

Die reduzierte spezifische Viskosität (RSV) dieser als Dispersionsstabilisatoren eingesetzten Copolymeren liegt in der Regel zwischen 0,01 und 0,1 dl/g, (bestimmt in 0,6 %iger Lösung in Toluol bei 25° C). Der entsprechende Vorzugsbereich beträgt bei den Copolymeren aus Maleinsäureanhydrid und Vinylalkyläther bzw. Vinylester 0,05 bis 1,0 dl/g und bei den Copolymeren aus Maleinsäureanhydrid und längerkettigem $\alpha$-Olefin 0,01 bis 0,1 dl/g. Das Molverhältnis zwischen Maleinsäureanhydrid und dem Vinylalkyläther bzw. Vinylester oder dem längerkettigen $\alpha$-Olefin liegt im allgemeinen zwischen 1:4 bis 1:1, vorzugsweise zwischen 1:2 bis 1:1 und insbesondere bei 1:1.

Als radikalisch wirksame Initiatoren kommen erfindungsgemäß solche in Betracht, die in der Monomerphase gut und in dem flüssigen Dispersionsmittel möglichst wenig löslich sind. Beispiele hierfür sind organische Peroxide, wie Di-tert.-butylperoxid, Dibenzoylperoxid, Bis(o-Methylbenzoyl)peroxid, tert.-Butylhydroperoxyd, Cumolhydroperoxid, Di-isopropylperoxidicarbonat, Cyclohexanonperoxid oder aliphatische Azoverbindungen, wie $\alpha,\alpha'$-Azodiisobuttersäurenitril, Azobis-cyanvaleriansäure, 1-1'-Azo-cyclo-hexan-1-1'-dicarbonsäurenitril- und Azodicarbonamid. Gegebenenfalls können auch entsprechende Redoxsysteme Verwendung finden. Die Menge an Initiator beträgt zumeist 0,01 bis 5, vorzugsweise 0,1 bis 2 Gew-% (bezogen auf die Gesamtmenge der Monomeren).

Als flüssige Dispersionsmittel zur Durchführung der erfindungsgemäßen Perlpolymerisation dienen vor allem solche organischen Verbindungen, die unter Normalbedingungen flüssig sind, einen Siedepunkt von oberhalb 60° C, vorzugsweise im Bereich von 85-300° C, aufweisen und welche vorzugsweise die Monomeren, das Polymere und vorzugsweise auch den Initiator unter den Polymerisationsbedingungen nicht oder jedenfalls nur spurenweise lösen. Gut geeignet sind beispielsweise Kohlenwasserstoffe mit 6 bis 20, vorzugsweise 8 bis 16 Kohlenstoffatomen, insbesondere Paraffine. Auch ein Gemisch von verschiedenen Verbindungen kann als Dispersionsmittel eingesetzt werden. Geeignete Kohlenwasserstoffe oder Kohlenwasserstoffgemische sind z.B. n-Hexan, n-Heptan, n-Octan, Cyclohexan, iso-Oktan, Benzinfraktionen mit Siedebereichen zwischen 90 und 170° C und Paraffinöl dünnflüssig (Deutsches Arzneibuch, 7. Ausgabe, DAB 7). Das Verhältnis der Monomerphase zur Dispersionsmittelphase kann in weiten Grenzen variieren, beispielsweise zwischen 1:1 bis 1:50, vorzugsweise 0,5:1 bis 1:15 (Gewichtsverhältnis).

Die Polymerisation der Monomeren gemäß Formel (IV) und gegebenenfalls weiterer damit copolymerisierbarer Monomeren erfolgt erfindungsgemäß in Anwesenheit der Verbindungen gemäß Formel (III), vorzugsweise eines oxäthylierten Alkohols und/oder einer oxäthylierten Carbonsäure, wobei diese Verbindungen in das entstehende Basispolymere eingebaut werden, überwiegend wohl durch Pfropfung.

Die Herstellung der Verbindungen gemäß Formel (III) ist bekannt und erfolgt durch Anlagerung von 1 bis 40, vorzugsweise 5 bis 35 Molen Alkylenoxyd, vorzugsweise Äthylenoxid, an ein Mol der entsprechenden Ausgangsverbindung

$$R_3 [ X H ]_n \quad (V),$$

in der $R_3$, X und n die Bedeutung gemäß Formel (III) haben, also beispielsweise an Alkohole, Amine, Carbonsäuren, Hydroxycarbonsäuren udgl. Die Obergrenze von 40 Mol ist dabei nicht kritisch, jedoch bringen wesentlich größere Mengen an Alkylenoxyd keine Vorteile.

Beispiele für Vertreter gemäß Formel (V) sind:

Einwertige, aliphatische Alkohole (Amine) wie Hexyl-, Ethyl-hexyl-, Heptyl-, Octyl-, Nonyl-, Decyl-, Undecyl-, Dodecyl-, Hexadecyl-, Octadecyl-, Eikosylalkohol(amin); einwertige Phenole (aromatische Amine), wie Hexylphenol (Hexylphenylamin), Nonylphenol (Nonylphenylamin), Dodecylphenol (Dodecylphenylamin); einwertige, aliphatische Carbonsäuren, wie Pentansäure, Hexansäure, Octansäure, Decansäure, Undecansäure, Dodecansäure, Tetradecansäure, Hexadecansäure, Heptadecansäure, Octadecansäure, Eicosansäure, Versaticsäure, Montansäure.

Die Verbindungen gemäß Formel (III) werden in einer solchen Menge eingesetzt, daß im Polymeren die weiter oben angegebenen Einbaumengen resultieren. Im allgemeinen betragen diese Mengen der Verbindungen (III), die im übrigen leicht experimentell zu bestimmen sind bis zu 30 Gew-%, vorzugsweise 1 bis 20 Gew-% und insbesondere 7 bis 15 Gew-%, bezogen auf das Monomere (IV) bzw. das Monomerengemisch.

Das erfindungsgemäße Verfahren wird zweckmäßigerweise in einem mit einer Rührvorrichtung verseh-

nen Reaktionsgefäß bei Temperaturen von zumeist 20 bis 150° C, vorzugsweise 60 bis 130° C durchgeführt. Die Teilchengröße des anfallenden Polymerisates wird in bekannter Weise durch die Rührgeschwindlgkeit und das Phasenverhältnis eingestellt. Besonders vorteilhaft ist die Verwendung eines senkrecht stehenden, zylindrischen Gefäßes mit flachem Boden, das mit einen koaxial angebrachten Rührer versehen ist, dessen Welle bis fast auf den Gefäßboden reicht. Das Reaktionsgefäß ist vorzugsweise vakuumfest und kann mit Rückflußkühler, Zulauftrichter, Gaseinleitungsrohr und Temperaturmeßgerät versehen werden. Die Beheizung und Kühlung des Gefäßes erfolgt im allgemeinen durch ein Flüssikeitsbad, z.B. ein Ölbad oder Wasserbad. Es ist vorteilhaft, das erfindungsgemäße Verfahren unter Ausschluß von Luftsauerstoff durchzuführen. Das Reaktionsgefäß wird daher vor Beginn mit einen inerten Gas, vorzugsweise Stickstoff, gespült.

Nach Beendigung der Polymerisationsreaktion wird das nicht umgesetzte Monomere aus dem Reaktionsgefäß entfernt, z.B. durch Verdampfen bei vermindertem Druck, vorzugsweise bei einem Druck von 0,1-15 Torr, oder durch Dekantieren, Filtrieren oder Absaugen des Überstandes. Anschließend wird das Polymerisat - falls erforderlich - mit leichtsiedenden organischen Lösungsmitteln, z.B. einem Kohlenwasserstoff, einem niederen Alkohol oder Aceton gewaschen und schließlich getrocknet. Die Trocknung des Polymerisats erfolgt bei einer Temperatur von zumeist 20 bis 100° C, vorzugsweise von 40-80° C; eine Trocknung unter vermindertem Druck ist dabei empfehlenswert.

Das nach dem erfindungsgemäßen Verfahren erhältliche Polymere besteht - falls unter den Bedingungen der Perlpolymerisation gearbeitet wurde - vor der vorzugsweise durchgeführten Verseifung aus überwiegend kugelförmigen Teilchen (Perlen), deren mittlere Teilchengröße im trockenen, ungequollenen Zustand 20 bis 800 μm, vorzugsweise 50 bis 300 μm beträgt und die vorzugsweise eine enge Teilchengrößenverteilung aufweisen. Das jeweilige Optimum der Teilchengröße hängt dabei vor allem von dem speziellen Einsatzgebiet ab.

Das nach dem erfindungsgemäßen Verfahren unmittelbar anfallende Polymerisat wird - wie erwähnt - vorzugsweise einer Hydrolyse unterworfen, wobei die cyclischen Carbonatgruppen geöffnet und unter Abspaltung von Kohlendioxid in sekundäre Hydroxylgruppen überführt werden. Die saure oder alkalische Hydrolyse kann dabei unter den bekannte Bedingungen erfolgen, wie etwa in J. Polym. Sci. 31, 237 (1958) beschrieben.

Die erfindungsgemäß erhältlichen Produkte eignen sich beispielsweise als Trägermaterialien für biologisch aktive Substanzen, als Adsorptionsmittel (Affinitätsharze), z.B. für die Blutfiltration oder bei der Gelchromatographie, für Formkörper (Folien, Fasern etc.) oder als Diagnostika in der Labormedizin (Teststreifen).

Bei Einsatz als Trägermaterial für biologisch aktive Substanzen wird dieser gegebenenfalls zunächst mit sogenannten Spacern umgesetzt. Als Spacer kommen erfindungsgemäß die hierfür bekannten homo- und hetero-bifunktionellen Verbindungen in Frage, deren zweite funktionelle Gruppe die Kopplung mit der zu fixierenden biologisch aktiven Substanz übernimmt (vgl. die DE-Patentschrift 2.421.789 und 2.552.510, sowie Ullmanns Encyclopädie der technischen Chemie, 4. Auflage, Bd. 10, S. 540 und "Characterization of Immobilized Biocatalysts", Verlag Chemie, Weinheim, 1979, S. 53).

Unter dem Begriff "biologisch aktive Substanzen" seien die bekannten in vivo oder in vitro wirksamen natürlichen oder künstlich hergestellten Stoffe verstanden, wie Enzyme, Aktivatoren, Inhibitoren, Antigene, Antikörper, Vitamine, Hormone, Effektoren, Antibiotika, Proteine udgl.. Der letztere Begriff umfaßt dabei auch Proteine mit bestimmten Nicht-Proteinsubstituenten wie Metallionen, Polysacchariden, Porphyringruppen, Adenindinucleotid, Ribonucleinsäure- oder Phospholipide . Auch Polypeptidfragmente, z.B. die aktiven Teile von Enzymmolekülen, fallen unter den Begriff biologisch aktive Substanzen.

Die Erfindung wird nachfolgend anhand von Beispielen näher erläutert.

Beispiel 1

a) In einem zylindrischen Gefäß mit Kreuzbalkenrührer, Rückflußkühler, Thermometer und Stickstoffeinleitrohr wurden 200 g Isooctan, 3,5 g oxäthyierter Stearylalkohol mit 25 Ethylenoxideinheiten und 0,75 g eines 1:1 Copolymerisates aus Maleinsäureanhydrid und Octadecen-(1) (RSV-Wert: 0,064 [dl/g] in 0,6 %iger Lösung in Toluol bei 26° C) vorgelegt. Nach Ausspülen des Gefäßes mit Stickstoff und Stickstoffüberlagerung wurde bei einer Rührgeschwindigkeit von 250 Upm 1 g Azodiisobuttersäurenitril in 150 g (= 110 ml) Vinylencarbonat gelöst, zugesetzt. Bei etwa 80° C setzte stürmische Wärmeentwicklung ein. Die Temperatur stieg innerhalb von Minuten schnell an, so daß es zu heftigem Rückfluß kam. Nachdem die Hauptreaktion abgelaufen war, wurde bei 95° C noch ca. 3 Stunden nachpolymerisiert. Nach beendeter Reaktion wurde das entstandene Polymerisat abgesaugt und mit Hexan mehrmals gewaschen. Nach Trocknung im Vakuumschrank bei 50° C wurden 120 g (= 80 % der Theorie) Polyvinylencarbonat-Pfropfpolymerisat erhalten. Es enthielt 2,0 Gew-% Äthoxy-Gruppen.

Der analytische Nachweis der aufgepfropften oxäthylierten Alkohole oder Säuren erfolgte durch Bestimmung der Oxäthylgruppe ($-C_2H_4O-$). Die Durchführung dieser analytischen Methode ist in der Zeitschrift "Industrial and Engineering Chemistry, Vol. 18, Nr. 8, August 1946, Seite 500-504, Analytical Edition" beschrieben.
b) 20 g des oben erhaltenen Polyvinylencarbonat-Pfropfpolymerisats wurden in 300 ml 5 n Natronlaugelösung eingerührt und 30 Minuten bei 95 °C verseift. Das Produkt ging dabei in Lösung. Nun wurden 2 1 Wasser zugesetzt und mit Salzsäure neutralisiert. Man filtrierte das ausgefallene Produkt über eine Nutsche. Nach Trocknung wurden 10 g pulverförmiges Material gewonnen.

Vergleichsversuch 1

Der Versuch 1 wurde unter denselben Versuchsbedingungen, jedoch ohne den Dispergator (Copolymerisat aus Maleinsäureanhydrid/Octadecen-(1) wiederholt.
Bei einsetzender Polymerisation mußte infolge zunehmender Verklumpung der Polymerisatteilchen die Rührung abgestellt werden. Nach beendeter 3-stündiger Polymerisation ohne Rührung, wurde der Reaktionskolben zerschlagen und das Polymerisat in einer Mühle zerkleinert. Die weitere Reinigungsoperation erfolgte wie in Beispiel 1 beschrieben. Bei der analytischen Prüfung wurden keine Oxäthylreste gefunden.

Vergleichsversuch 2

a) In der Versuchsordnung gemäß Beispiel 1 wurden in 970 g Isooctan 1,5 g eines Copolymerisates aus Maleinsäureanhydrid und Octadecen-(1) vorgelegt. Nun wurden in 450 g Vinylencarbonat 3 g Azodiisobuttersäurenitril gelöst und der obigen Mischung zugesetzt. Die Badtemperatur wurde langsam auf 80 °C erhöht. Bei einsetzender Polymerisation stieg die Innentemperatur rasch auf 105-110° (Rückfluß) an. Es wurde bei 95° 3 Stunden nachpolymerisiert. Ein Verklumpen des Polymerisats trat nicht auf, da der erfindungsgemäße Dispersionsstabilisator anwesend war. Nach Aufarbeitung des Polymerisates wurden 380 g Polyvinylencarbonat erhalten.
b) 40 g des vorstehend erhaltenen Polyvinylencarbonats wurden in einem Gemisch von 200 ml Methanol und 100 ml 2 n-Natronlauge 3 Stunden bei Rückflußtemperatur gerührt. Die Natronlauge wurde mit verdünnter Salzsäure neutralisiert, mit Wasser mehrmals und dann mit Aceton nachgewaschen. Nach Trocknen wurden 24 g Polyhydroxymethylen erhalten.

Beispiele 2 - 4

a) Gemäß der in Beispiel 1 beschriebenen Versuchsordnung wurden die in der Tabelle 1 aufgeführten Mengen an Isooctan und an oxyäthyliertem Stearylalkohol sowie des 1:1 Copolymerisates aus Maleinsäureanhydrid und Octadecen-(1) zugesetzt. Anschließend löste man Azodiisobuttersäurenitril in Vinylencarbonat und gab diese Lösung zu. Der Polymerisationsverlauf und die Aufarbeitung erfolgte nach der in Beispiel 1 beschriebenen Weise.
b) 20 g des nach Beispiel 3 erhaltenen gepfropften Polyvinylencarbonates wurden in einer Lösung von 100 ml Methanol und 50 ml 2 n-Natronlauge 3 Stunden bei Rückflußtemperatur unter Rühren hydrolysiert. Anschließend wurde die Natronlauge mit Essigsäure neutralisiert. Der Niederschlag wurde abgesaugt, mit Wasser mehrmals gewaschen und dann mit Aceton behandelt. Nach Trocknen wurden 16 g Produkt erhalten.

Tabelle 1

| Beispiele | Menge Dispersionsmittel (g) | Menge Vinylencarbonat (g) | Oxäthylierter Stearylalkohol mit 25 Äthylenoxid-Einheiten (g) | Dispersionsstabilisator (g) | Azodiisobuttersäurenitril (g) | Einbau von Äthoxygruppen (Theorie, Gew-%) | Äthoxygruppen (Gew-% gefunden) [Einbaurate in %] | Ausbeute |
|---|---|---|---|---|---|---|---|---|
| 1 | 200 | 150 | 3,5 | 0,75 | 1 | 2,3 | 2,2 [87] | 121 g ≙ 76,5 % |
| 2 | 200 | 150 | 9,0 | 1,0 | 1 | 5,6 | 5,5 [95] | 117 g ≙ 76,1 % |
| 3 | 200 | 150 | 30 | 0,3 | 1 | 16,6 | 20 [96] | 112 g ≙ 62,2 % |
| 4 | 970 | 450 | 45 | 1,0 | 3 | 9,1 | 7,8 [91] | 406 ≙ 82 % |
| Vergleichsversuch 1 | 200 | 150 | 3,5 | 0 | 1 | - | 0 | |
| Vergleichsverusch 2 | 970 | 450 | 0 | 1,5 | 3 | - | - | 380 g ≙ 84 % |

## Beispiel 5

Gemäß Beispiel 1 wurden 200 g Isooctan, 6 g oxäthyliertes Octanol ($C_8H_{17}$-O-($CH_2$-$CH_2$-O)$_{10}$-H), 1 g eines 1:1 Copolymerisates aus Maleinsäureanhydrid und Vinyloctadecylether (RSV-Wert = 0,22 [dl/g] in

0,6-%iger Lösung in Toluol bei 26° C) vorgelegt. Dann wurden 1 g Azodiisobuttersäurenitril in 150 g Vinylencarbonat zugesetzt. Bei 70-80° setzte die Polymerisation ein. Die gesamte Polymerisationsdauer betrug ca. 3 Stunden. Das Polymerisat wurde abgesaugt, mit Hexan und Methanol mehrmals gewaschen. Nach Trocknen wurden 115 g Produkt erhalten.

Beispiel 6

Gemäß Beispiel 1 wurden 200 g Isooctan, 45 g oxäthylierter Talgfettalkohol mit 25 Ethylenoxideinheiten und 1,5 g eines 1:1 Copolymerisates aus Maleinsäureanhydrid und Octadecen(1) (RSV-Wert: 0,064 [dl/g] in 0,6 %iger Lösung in Toluol bei 26° C) vorgelegt. Dann wurden 1 g Azodiisobuttersäurenitril in 150 g Vinylencarbonat zugesetzt. Die Polymerisationsdauer betrug 3 Stunden bei 95° C. Nach Absaugen und Auswaschen mit Hexan und Methanol wurden nach dem Trocknen 150 g Polymerisat erhalten (= 77 % Ausbeute).

Beispiel 7

Gemäß Beispiel 1 wurden 300 ml dünnflüssiges Paraffinöl, 15 g oxäthylierter Talgfettalkohol mit 25 Ethylenoxideinheiten und 1,5 g eines 1:1 Copolymerisates aus Maleinsäureanhydrid und Octadecen-(1), (RSV-Wert: 0,064 [dl/g] in 0,6 %iger toluolischer Lösung bei 26° C) vorgelegt. Dann wurden 1,5 g Azodiisobuttersäurenitril und 3 g Divinylenharnstoff in 150 g Vinylencarbonat gelöst, zugesetzt und unter Rühren langsam hochgeheizt. Die Temperatur von 80° C wurde 1 Stunde gehalten, dann die Temperatur auf 95° C erhöht und 5 Stunden polymerisiert.

Das perlförmige Produkt wurde abgesaugt, 3 mal mit je 150 ml Hexan und je 2 mal mit 200 ml Methanol ausgerührt. Nach Absaugen und Trocknen im Vakuumschrank bei 50° C wurden 83 g (45 %) Produkt gewonnen.

Beispiel 8

Der Versuch wurde analog Beispiel 6 durchgeführt mit der Maßgabe, daß anstelle von 15 g des oxäthylierten Talgfettalkohols 30 g dieser Substanz eingesetzt wurden.

Die Ausbeute betrug 120 g (= 65 %).

Die Verseifung der gepfropften Polyvinylencarbonatgruppen erfolgte gemäß der in den Beispielen 2-4 unter b) beschriebenen Methode.

## Patentansprüche

1. Polymerisat, im wesentlichen bestehend aus den Einheiten

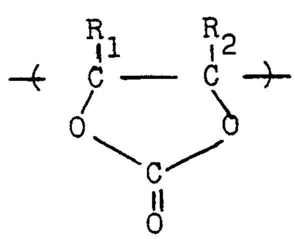 und/oder (I)

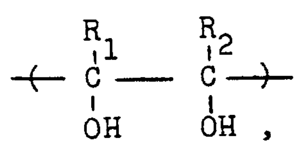 , (II)

worin $R_1$ und/oder $R_2$ Wasserstoff oder einen einwertigen Kohlenwasserstoffrest mit bis zu 8 Kohlenstoffatomen bedeuten,
und gegebenenfalls kleiner Mengen von weiteren Monomer-Einheiten, dadurch gekennzeichnet, daß es

noch Einheiten kovalent gebunden enthält, die sich ableiten von mindestens einer Verbindung der Formel

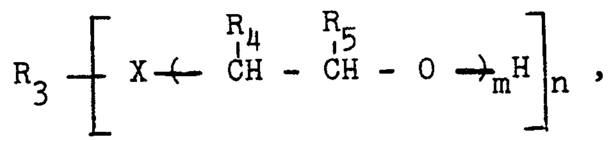

(III)

worin bedeuten:

$R_3$ = Kohlenwasserstoffrest mit 4 bis 30 Kohlenstoff-Atomen;
X = -O-, -NH- und/oder -COO-;
$R_4$, $R_5$ = Wasserstoff oder einen einwertigen Kohlenwasserstoffrest mit 1 bis 8 Kohlenstoff-Atomen, mit der Maßgabe, daß mindestens einer der Reste $R_4$, $R_5$ Wasserstoff ist;
m = ganze Zahl von 1 bis 40;
n = ganze Zahl von 1 bis 4.

**2.** Polymerisat nach Anspruch 1, dadurch gekennzeichnet, daß die Menge an (II) mindestens 95 Gew-%, bezogen auf die Gesamtmenge an (I) und (II), beträgt.

**3.** Polymerisat nach Anspruch 1 und/oder 2, dadurch gekennzeichnet, daß die Menge an Einheiten gemäß Formel (III) 1 bis 20 Gew-%, bezogen auf das Basispolymere, beträgt.

**4.** Polymerisat nach einem oder mehreren der Ansprüche 1 bis 3, dadurch gekennzeichnet, daß $R_1$ und $R_2$ in (I) bzw. (II) für Wasserstoff stehen.

**5.** Polymerisat nach einem oder mehreren der Ansprüche 1 bis 4, dadurch gekennzeichnet, daß $R_3$ für einen Alkylrest mit 4 bis 30 Kohlenstoffatomen, X für -O- und/oder -COO-, $R_4$/$R_5$ für Wasserstoff, m für 5 bis 35 und n für 1 oder 2 stehen.

**6.** Polymerisat nach einem oder mehreren der Ansprüche 1 bis 5, dadurch gekennzeichnet, daß $R_3$ für einen Alkylrest mit 12 bis 20 Kohlenstoffatomen und X für -O- und/oder -COO- stehen.

**7.** Polymerisat nach Anspruch 6, dadurch gekennzeichnet, daß X nur für -O- steht.

**8.** Verfahren zur Herstellung der Polymerisate nach einem oder mehreren der Ansprüche 1 bis 7 durch Polymerisation von Verbindungen der Formel

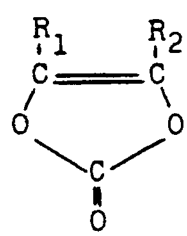

(IV)

worin $R_1$ und $R_2$ die obige Redeutung haben, und gegebenenfalls kleiner Mengen weiterer damit copolymerisierbarer Monomere, wobei die Polymerisation in einem flüssigen Dispersionsmittel, in Gegenwart eines radikalisch wirksamen Initiators und eines Dispersionsstabilisators durchgeführt wird, und gegebenenfalls Hydrolyse der Cyclocarbonatringe des so erhaltenen Produkts, dadurch gekennzeichnet, daß die Polymerisation in Gegenwart von mindestens einer Verbindung gemäß Formel (III), in der $R_3$ bis $R_5$, X, m und n die obige Bedeutung haben, erfolgt und daß als Dispersionsstabilisator ein Copolymerisat aus Maleinsäureanhydrid und einem Vinylalkyläther und/oder einem Vinylester und/oder einem längerkettigen $\alpha$-Olefin verwendet wird.

**9.** Verfahren nach Anspruch 8, dadurch gekennzeichnet, daß der eingesetzte Dispersionsstabilisator ein Copolymerisat aus Maleinsäureanhydrid und einem Vinylalkyläther mit 6 bis 30 C-Atomen im Alkylrest

und/oder einem Vinylester mit 6 bis 30 C-Atomen in der Acylgruppe und/oder einem längerkettigen α-Olefin mit 8 bis 30 C-Atomen darstellt.

**10.** Verfahren nach Anspruch 8 und/oder 9, dadurch gekennzeichnet, daß der Dispersionsstabilisator Maleinsäureanhydrid und die Comonomeren im Verhältnis von etwa 1:1 enthält.

**11.** Verfahren nach einem oder mehreren der Ansprüche 8 bis 10, dadurch gekennzeichnet, daß der Dispersionsstabilisator in Mengen von 0,001 bis 10 Gew-%, bezogen auf die Gesamtmenge an Monomeren, verwendet wird.

**12.** Verfahren nach einem oder mehreren der Ansprüche 8 bis 11, dadurch gekennzeichnet, daß der Vinylalkyläther Vinylstearyläther und das längerkettige α-Olefin Octadecen(1) ist.

**13.** Verfahren nach einem oder mehreren der Ansprüche 8 bis 12, dadurch gekennzeichnet, daß als Verbindungen der Formel (III) oxäthylierte Alkohole und/oder oxäthylierte Carbonsäuren in Mengen von 1 bis 30 Gew-%, bezogen auf die Gesamtmenge an Monomeren, eingesetzt werden.

**14.** Verfahren nach Anspruch 13, dadurch gekennzeichnet, daß es sich bei den Alkoholen um einwertige, primäre Alkohole und bei den Carbonsäuren um einbasische, primäre Carbonsäuren handelt und der Kohlenwasserstoffrest in beiden Fällen ein Alkylrest mit 4 bis 30 Kohlenstoff-Atomen ist.

**15.** Verfahren nach einem oder mehreren der Ansprüche 8 bis 14, dadurch gekennzeichnet, daß als flüssige Dispersionsmittel Kohlenwasserstoffe mit 6 bis 20 C-Atomen oder Paraffinöl dünnflüssig eingesetzt werden.

**16.** Verwendung der Polymerisate nach einem oder mehreren der Ansprüche 1 bis 7, gegebenenfalls nach Umsatz mit Spacern, zur Herstellung trägergebundener, biologisch aktiver Substanzen, als Adsorptionsmittel oder zur Herstellung von Formkörpern.

**17.** Verwendung nach Anspruch 16, dadurch gekennzeichnet, daß die biologisch aktiven Substanzen Enzyme sind.

**Patentansprüche für folgenden Vertragsstaat: AT**

**1.** Verfahren zur Herstellung eines Polymerisats, im wesentlichen bestehend aus den Einheiten

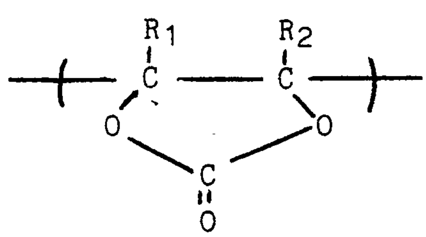 und/oder (I)

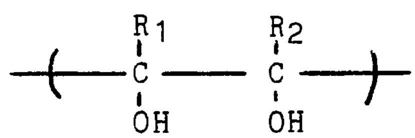 (II)

worin $R_1$ und/oder $R_2$ Wasserstoff oder einen einwertigen Kohlenwasserstoffrest mit bis zu 8 Kohlenstoffatomen bedeuten,
und gegebenenfalls kleiner Mengen von weiteren Monomer-Einheiten, durch Polymerisation von Verbindungen der Formel

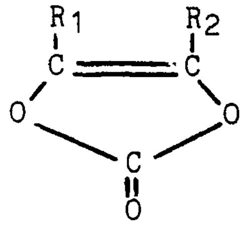

(IV)

worin $R_1$ und $R_2$ die obige Bedeutung haben, und gegebenenfalls kleiner Mengen weiterer damit copolymerisierbarer Monomere, wobei die Polymerisation in einem flüssigen Dispersionsmittel in Gegenwart eines radikalisch wirksamen Initiators und eines Dispersionsstabilisators durchgeführt wird, und gegebenenfalls Hydrolyse der Cyclooarbonatringe des so erhaltenen Produkts, dadurch gekennzeichnet, daß die Polymerisation in Gegenwart von mindestens einer Verbindung der Formel

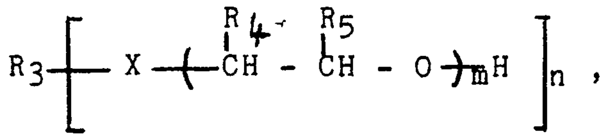

(III)

worin bedeuten:

$R_3$ = Kohlenwasserstoffrest mit 4 bis 30 Kohlenstoffatomen;
X = -O-, -NH- und/oder -COO-;
$R_4$, $R_5$ = Wasserstoff oder einen einwertigen Kohlenwasserstoffrest mit 1 bis 8 Kohlenstoffatomen, mit der Maßgabe, daß mindestens einer der Reste $R_4$, $R_5$ Wasserstoff ist;
m = ganze Zahl von 1 bis 40;
n = ganze Zahl von 1 bis 4

erfolgt, wobei die sich von der Formel (III) ableitenden Einheiten kovalent gebunden werden, und daß als Dispersionsstabilisator ein Copolymerisat aus Maleinsäureanhydrid und einem Vinylalkyläther und/oder einem Vinylester und/oder einem längerkettigen $\alpha$-Olefin verwendet wird.

2. Verfahren nach Anspruch 1, dadurch gekennzeichnet, daß Polymerisate erhalten werden, bei denen die Menge an (II) mindestens 95 Gew.-%, bezogen auf die Gesamtmenge an (I) und (II), beträgt.

3. Verfahren nach Anspruch 1 oder 2, dadurch gekennzeichnet, daß die Menge an Einheiten gemäß Formel (III) 1 bis 20 Gew.-%, bezogen auf das Basispolymere, beträgt.

4. Verfahren nach einem oder mehreren der Ansprüche 1 bis 3, dadurch gekennzeichnet, daß $R_1$ und $R_2$ in (I) bzw. (II) für Wasserstoff stehen.

5. Verfahren nach einem oder mehreren der Ansprüche 1 bis 4, dadurch gekennzeichnet, daß $R_3$ für einen Alkylrest mit 4 bis 30 Kohlenstoffatomen, X für -O- und/oder -COO-, $R_4/R_5$ für Wasserstoff, m für 5 bis 35 und n für 1 oder 2 stehen.

6. Verfahren nach einem oder mehreren der Ansprüche 1 bis 5, dadurch gekennzeichnet, daß $R_3$ für einen Alkylrest mit 12 bis 20 Kohlenstoffatomen und X für -O- und/oder -COO-stehen.

7. Verfahren nach Anspruch 6, dadurch gekennzeichnet, daß X nur für -O- steht.

8. Verfahren nach einem oder mehreren der Ansprüche 1 bis 7, dadurch gekennzeichnet, daß der eingesetzte Dispersionsstabilisator ein Copolymerisat aus Maleinsäureanhydrid und einem Vinylalkyläther mit 6 bis 30 C-Atomen im Alkylrest und/oder einem Vinylester mit 6 bis 30 C-Atomen in der Acylgruppe und/oder einem längerkettigen $\alpha$-Olefin mit 8 bis 30 C-Atomen darstellt.

9. Verfahren nach einem oder mehreren der Ansprüche 1 bis 8, dadurch gekennzeichnet, daß der Dispersionsstabilisator Maleinsäureanhydrid und die Comonomeren im Verhältnis von etwa 1:1 enthält.

**10.** Verfahren nach einem oder mehreren der Ansprüche 1 bis 9, dadurch gekennzeichnet, daß der Dispersionsstabilisator in Mengen von 0,001 bis 10 Gew.-%, bezogen auf die Gesamtmenge an Monomeren, verwendet wird.

**11.** Verfahren nach einem oder mehreren der Ansprüche 1 bis 10, dadurch gekennzeichnet, daß der Vinylalkyläther Vinylstearyläther und das längerkettige α-Olefin Octadecen(1) ist.

**12.** Verfahren nach einem oder mehreren der Ansprüche 1 bis 11, dadurch gekennzeichnet, daß als Verbindungen der Formel (III) oxäthylierte Alkohole und/oder oxäthylierte Carbonsäuren in Mengen von 1 bis 30 Gew.-%, bezogen auf die Gesamtmenge an Monomeren, eingesetzt werden.

**13.** Verfahren nach Anspruch 12, dadurch gekennzeichnet, daß es sich bei den Alkoholen um einwertige, primäre Alkohole und bei den Carbonsäuren um einbasische, primäre Carbonsäuren handelt und der Kohlenwasserstoffrest in beiden Fällen ein Alkylrest mit 4 bis 30 Kohlenstoffatomen ist.

**14.** Verfahren nach einem oder mehreren der Ansprüche 1 bis 13, dadurch gekennzeichnet, daß als flüssige Dispersionsmittel Kohlenwasserstoffe mit 6 bis 20 C-Atomen oder Paraffinöl dünnflüssig eingesetzt werden.

**15.** Verwendung der Polymerisate, hergestellt nach einem oder mehreren der Ansprüche 1 bis 14, gegebenenfalls nach Umsatz mit Spacern, zur Herstellung trägergebundener, biologisch aktiver Substanzen, als Adsorptionsmittel oder zur Herstellung von Formkörpern.

**16.** Verwendung nach Anspruch 15, dadurch gekennzeichnet, daß die biologisch aktiven Substanzen Enzyme sind.

**Claims**

**1.** A polymer substantially consisting of the units

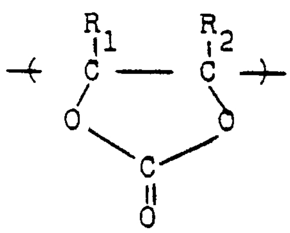

(I)

and/or

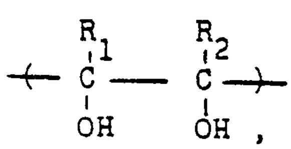

(II)

in which $R_1$ and/or $R_2$ denote hydrogen or a monovalent hydrocarbon radical with up to 8 carbon atoms, and, if appropriate, small amounts of other monomer units, which also contains covalently bonded units which are derived from at least one compound of the formula

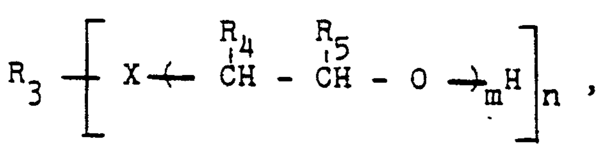

(III)

in which:

| | |
|---|---|
| $R_3$ | denotes a hydrocarbon radical with 4 to 30 carbon atoms; |
| X | denotes -O-, -NH- and/or -COO-; |
| $R_4$ and $R_5$ | denote hydrogen or a monovalent hydrocarbon radical with 1 to 8 carbon atoms, with the proviso that at least one of the radicals $R_4$ or $R_5$ is hydrogen; |
| m | denotes an integer from 1 to 40; and |
| n | denotes an integer from 1 to 4. |

**2.** A polymer as claimed in claim 1, wherein the amount of (II) is at least 95% by weight, based on the total amount of (I) and (II).

**3.** A polymer as claimed in claims 1 and/or 2, wherein the amount of units of the formula (III) is 1 to 20% by weight, based on the base polymer.

**4.** A polymer as claimed in one or more of claims 1 to 3, wherein $R_1$ and $R_2$ in (I) or, respectively (II), represent hydrogen.

**5.** A polymer as claimed in one or more of claims 1 to 4, wherein $R_3$ represents an alkyl radical with 4 to 30 carbon atoms, X represents -O- and/or -COO-, $R_4$ and $R_5$ represent hydrogen, m represents 5 to 35 and n represents 1 or 2.

**6.** A polymer as claimed in one or more of claims 1 to 5, wherein $R_3$ represents an alkyl radical with 12 to 20 carbon atoms and X represents -O- and/or -COO-.

**7.** A polymer as claimed in claim 6, wherein X represents only -O-.

**8.** A process for the preparation of a polymer as claimed in one or more of claims 1 to 7 by polymerization of a compound of the formula

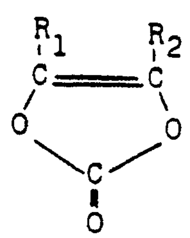

(IV)

in which $R_1$ and $R_2$ have the above meaning, and, if appropriate, small amounts of other monomers copolymerizable with these compounds, the polymerization being carried out in a liquid dispersant in the presence of a free radical initiator and a dispersion stabilizer, and, if appropriate, hydrolysis of the cyclocarbonate rings of the resulting product, which comprises carrying out the polymerization in the presence of at least one compound of the formula (III), in which $R_3$ to $R_5$, X, m and n have the above meaning, and using, as the dispersion stabilizer, a copolymer of maleic anhydride and a vinyl alkyl ether and/or a vinyl ester and/or a longer chain $\alpha$-olefin.

**9.** The process as claimed in claim 8, wherein the dispersion stabilizer employed is a copolymer of maleic anhydride and a vinyl alkyl ether with 6 to 30 carbon atoms in the alkyl radical and/or a vinyl ester with 6 to 30 carbon atoms in the acyl group and/or a longer-chain $\alpha$-olefin with 8 to 30 carbon atoms.

**10.** The process as claimed in claim 8 and/or 9, wherein the dispersion stabilizer contains maleic anhydride and the comonomers in a ratio of about 1:1.

**11.** The process as claimed in one or more of claims 8 to 10, wherein the dispersion stabilizer is used in an amount of 0.001 to 10% by weight, based on the total amount of monomers.

**12.** The process as claimed in one or more of claims 8 to 11, wherein the vinyl alkyl ether is vinyl stearyl ether and the longer-chain a-olefin is octadec-1-ene.

13. The process as claimed in one or more of claims 8 to 12, wherein an oxyethylated alcohol and/or oxyethylated carboxylic acid in an amount of 1 to 30% by weight, based on the total amount of monomers, are employed as the compounds of the formula (III).

14. The process as claimed in claim 13, wherein the alcohol is a monohydric primary alcohol and the carboxylic acid is a monobasic primary carboxylic acid, and the hydrocarbon radical in both cases is an alkyl radical with 4 to 30 carbon atoms.

15. The process as claimed in one or more of claims 8 to 14, wherein a hydrocarbon with 6 to 20 carbon atoms or light liquid paraffin is employed as the liquid dispersant.

16. The use of a polymer as claimed in one or more of claims 1 to 7, if appropriate after reaction with a spacer, for the preparation of a carrier-bound biologically active substance, as an adsorbent or for the production of a shaped article.

17. The use as claimed in claim 16, wherein the biologically active substance is an enzyme.

**Claims for the following Contracting State: AT**

1. A process for the preparation of a polymer substantially consisting of the units

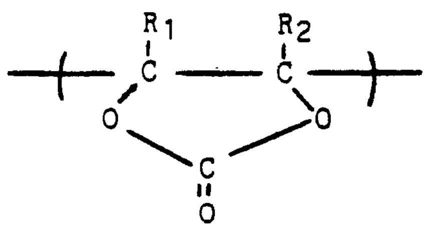

(I)

and/or

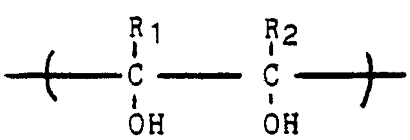

(II)

in which $R_1$ and/or $R_2$ denote hydrogen or a monovalent hydrocarbon radical with up to 8 carbon atoms, and, if appropriate, small amounts of other monomer units, by polymerization of a compound of the formula

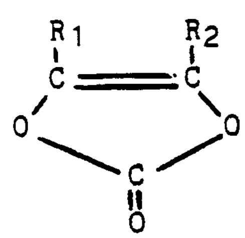

(IV)

in which $R_1$ and $R_2$ have the above meaning, and, if appropriate, small amounts of other monomers copolymerizable with these compounds, the polymerization being carried out in a liquid dispersant in the presence of a free radical initiator and a dispersion stabilizer, and, if appropriate, hydrolysis of the cyclocarbonate rings of the resulting product, which comprises carrying out the polymerization in the presence of at least one compound of the formula

$$R_3\left[X-\left(\overset{R_4}{\underset{|}{C}}H-\overset{R_5}{\underset{|}{C}}H-O\right)_m H\right]_n, \qquad (IV)$$

in which:

| | |
|---|---|
| $R_3$ | denotes a hydrocarbon radical with 4 to 30 carbon atoms; |
| X | denotes -O-, -NH- and/or -COO-; |
| $R_4$ and $R_5$ | denote hydrogen or a monovalent hydrocarbon radical with 1 to 8 carbon atoms, with the proviso that at least one of the radicals $R_4$ or $R_5$ is hydrogen; |
| m | denotes an integer from 1 to 40; and |
| n | denotes an integer from 1 to 4, |

the units derived from the formula (III) being bonded covalently, and comprises using, as the dispersion stabilizer, a copolymer of maleic anhydride and a vinyl alkyl ether and/or a vinyl ester and/or a longer chain α-olefin.

**2.** The process as claimed in claim 1, wherein polymers are obtained in which the amount of (II) is at least 95% by weight, based on the total amount of (I) and (II).

**3.** The process as claimed in claim 1 or 2, wherein the amount of units of the formula (III) is 1 to 20% by weight based on the base polymer.

**4.** The process as claimed in one or more of claims 1 to 3, wherein $R_1$ and $R_2$ in (I) or, respectively (II), represent hydrogen.

**5.** The process as claimed in one or more of claims 1 to 4, wherein $R_3$ represents an alkyl radical with 4 to 30 carbon atoms, X represents -O- and/or -COO-, $R_4$ and $R_5$ represent hydrogen, m represents 5 to 35 and n represents 1 or 2.

**6.** The process as claimed in one or more of claims 1 to 5, wherein $R_3$ represents an alkyl radical with 12 to 20 carbon atoms and X represents -O- and/or -COO-.

**7.** The process as claimed in claim 6, wherein X represents only -O-.

**8.** The process as claimed in one or more of claims 1 to 7, wherein the, dispersion stabilizer employed is a copolymer of maleic anhydride and a vinyl alkyl ether with 6 to 30 carbon atoms in the alkyl radical and/or a vinyl ester with 6 to 30 carbon atoms in the acyl group and/or a longer-chain α-olefin with 8 to 30 carbon atoms.

**9.** The process as claimed in one or more of claims 1 to 8, wherein the dispersion stabilizer contains maleic anhydride and the comonomers in a ratio of about 1:1.

**10.** The process as claimed in one or more of claims 1 to 9, wherein the dispersion stabilizer is used in an amount of 0.001 to 10% by weight, based on the total amount of monomers.

**11.** The process as claimed in one or more of claims 1 to 10, wherein the vinyl alkyl ether is vinyl stearyl ether and the longer-chain α-olefin is octadec-1-ene.

**12.** The process as claimed in one or more of claims 1 to 11, wherein an oxyethylated alcohol and/or oxyethylated carboxylic acid in an amount of 1 to 30% by weight, based on the total amount of monomers, are employed as the compounds of the formula (III).

**13.** The process as claimed in claim 12, wherein the alcohol is a monohydric primary alcohol and the carboxylic acid is a monobasic primary carboxylic acid, and the hydrocarbon radical in both cases is an alkyl radical with 4 to 30 carbon atoms.

**14.** The process as claimed in one or more of claims 1 to 13, wherein a hydrocarbon with 6 to 20 carbon

atoms or light liquid paraffin is employed as the liquid dispersant.

**15.** The use of a polymer prepared as claimed in one or more of claims 1 to 14, if appropriate after reaction with a spacer, for the preparation of a carrier-bound biologically active substance, as an adsorbent or for the production of a shaped article.

**16.** The use as claimed in claim 15, wherein the biologically active substance is an enzyme.

**Revendications**

**1.** Polymère substantiellement constitué de motifs de formule I :

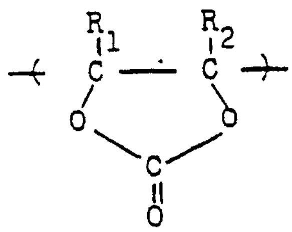

(I)

et/ou de motifs de formule II :

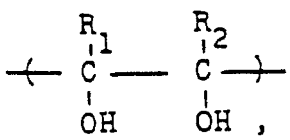

(II),

formules dans lesquelles $R_1$ et/ou $R_2$ représentent l'hydrogène ou un radical hydrocarboné univalent contenant au plus 8 atomes de carbone,
et éventuellement de petites quantités d'autres motifs monomères, polymère caractérisé en ce qu'il contient en outre des motifs, liés par covalence, qui dérivent d'au moins un composé répondant à la formule III :

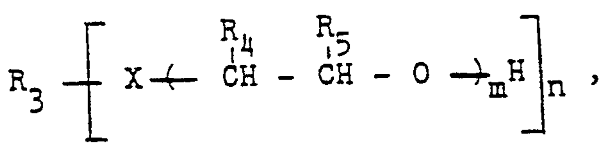

(III)

dans laquelle :

| | |
|---|---|
| $R_3$ | représente un radical hydrocarboné contenant de 4 à 30 atomes de carbone, |
| X | représente -O-, -NH- et/ou -COO-, |
| $R_4$ et $R_5$ | représentent chacun l'hydrogène ou un radical hydrocarboné univalent contenant de 1 à 8 atomes de carbone, avec la condition qu'au moins un des symboles $R_4$ et $R_5$ représente l'hydrogène, |
| m | désigne un nombre entier de 1 à 40, et |
| n | désigne un nombre entier de 1 à 4. |

**2.** Polymère selon la revendication 1 caractérisé en ce que la quantité de (II) est d'au moins 95% en poids par rapport à la quantité totale de (I) et (II).

**3.** Polymère selon l'une des revendications 1 et 2, caractérisé en ce que la quantité de motifs provenant de (III) est comprise entre 1 et 20% en poids par rapport au polymère de base.

4. Polymère selon l'une quelconque des revendications 1 à 3, caractérisé en ce que $R_1$ et $R_2$, dans (I) ou (II), représentent chacun l'hydrogène.

5. Polymère selon l'une quelconque des revendications 1 à 4, caractérisé en ce que $R_3$ représente un alkyle contenant de 4 à 30 atomes de carbone, X représente -O- et/ou -COO-, $R_4$ et $R_5$ représentent chacun l'hydrogène, m désigne un nombre de 5 à 35, et n est égal à 1 ou à 2.

6. Polymère selon l'une quelconque des revendications 1 à 5, caractérisé en ce que $R_3$ représente un alkyle contenant 12 à 20 atomes de carbone, et X représente -O- et/ou -COO-.

7. Polymère selon la revendication 6 caractérisé en ce que X ne représente que -O-.

8. Procédé pour préparer des polymères selon l'une quelconque des revendications 1 à 7, par polymérisation de composés répondant à la formule IV :

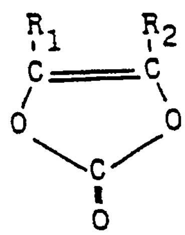

(IV)

dans laquelle $R_1$ et $R_2$ ont les significations qui leur ont été données ci-dessus, et éventuellement de petites quantités d'autres monomères copolymérisables avec les précédents, la polymérisation étant effectuée dans un milieu de dispersion liquide, en présence d'un amorceur radicalaire et/ou d'un stabilisant de dispersion, et éventuellement hydrolyse des cycles cyclocarbonates du produit ainsi obtenu, procédé caractérisé en ce qu'on effectue la polymérisation en présence d'au moins un composé répondant à la formule III dans laquelle $R_3$ à $R_5$, X, m et n ont les significations qui leur ont été données ci-dessus, et en ce qu'on utilise, comme stabilisant de dispersion, un copolymère de l'anhydride maléique et d'un oxyde de vinyle et d'alkyle et/ou d'un ester vinylique et/ou d'une $\alpha$-oléfine à longue chaîne.

9. Procédé selon la revendication 8 caractérisé en ce que le stabilisant de dispersion utilisé est un copolymère de l'anhydride maléique et d'un oxyde de vinyle et d'alkyle dont le radical alkyle contient de 6 à 30 atomes de carbone et/ou d'un ester vinylique dont le radical acyle contient de 6 à 30 atomes de carbone et/ou d'une $\alpha$-oléfine à longue chaîne qui contient de 8 à 30 atomes de carbone.

10. Procédé selon l'une des revendications 8 et 9, caractérisé en ce que le stabilisant de dispersion contient l'anhydride maléique et les comonomères dans un rapport d'environ 1 : 1.

11. Procédé selon l'une quelconque des revendications 8 à 10, caractérisé en ce qu'on utilise le stabilisant de dispersion en une quantité de 0,001 à 10% en poids par rapport à la quantité totale des monomères.

12. Procédé selon l'une quelconque des revendications 8 à 11, caractérisé en ce que l'oxyde de vinyle et d'alkyle est l'oxyde de vinyle et de stéaryle, et l'$\alpha$-oléfine à longue chaîne est l'octadécène-1.

13. Procédé selon l'une quelconque des revendications 8 à 12, caractérisé en ce qu'on utilise, comme composés de formule III, des alcools éthoxylés et/ou des acides carboxyliques éthoxylés, en des quantités de 1 à 30% en poids par rapport à la quantité totale des monomères.

14. Procédé selon la revendication 13 caractérisé en ce que les alcools sont des monoalcools primaires, les acides carboxyliques sont des monoacides carboxyliques primaires, et, dans les deux cas, le radical hydrocarboné est un alkyle contenant de 4 à 30 atomes de carbone.

15. Procédé selon l'une quelconque des revendications 8 à 14, caractérisé en qu'on utilise, comme milieux

de dispersion liquides, des hydrocarbures contenant de 6 à 20 atomes de carbone ou une huile de paraffine très fluide.

**16.** Application des polymères selon l'une quelconque des revendications 1 à 7, éventuellement après réaction avec des "écarteurs", à la fabrication de substances biologiquement actives liées à des supports, comme agents d'adsorption ou pour la fabrication d'objets moulés.

**17.** Application selon la revendication 16 caractérisée en ce que les substances biologiquement actives sont des enzymes.

**Revendications pour l'Etat contractant suivant: AT**

**1.** Procédé pour préparer un polymère substantiellement constitué de motifs de formule I :

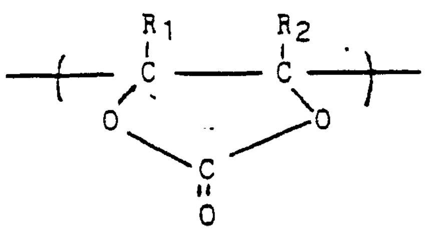

(I)

et/ou de motifs de formule II :

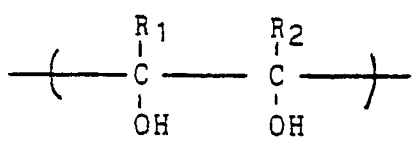

(II),

formules dans lesquelles $R_1$ et/ou $R_2$ représentent l'hydrogène ou un radical hydrocarboné univalent qui contient au plus 8 atomes de carbone,
et éventuellement de petites quantités d'autres motifs monomères, par polymérisation de composés répondant à la formule IV :

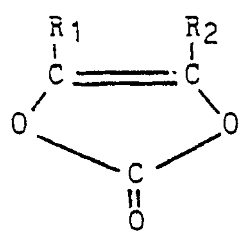

(IV)

dans laquelle $R_1$ et $R_2$ ont les significations qui leur ont été données ci-dessus, et éventuellement de petites quantités d'autres monomères copolymérisables avec les précédents, la polymérisation étant effectuée dans un milieu de dispersion liquide, en présence d'un amorceur à activité radicalaire et d'un stabilisant de dispersion, et éventuellement hydrolyse des cycles cyclocarbonates des produits ainsi obtenus, procédé caractérisé en ce qu'on effectue la polymérisation en présence d'au moins un composé répondant à la formule III :

$$R_3\left[X\left(\overset{R_4}{\underset{|}{C}H}-\overset{R_5}{\underset{|}{C}H}-O\right)_m H\right]_n \quad \text{(III)}$$

dans laquelle :

| | |
|---|---|
| $R_3$ | représente un radical hydrocarboné contenant de 4 à 30 atomes de carbone, |
| X | représente -O-, -NH- et/ou -COO-, |
| $R_4$ et $R_5$ | représentent chacun l'hydrogène ou un radical hydrocarboné univalent contenant de 1 à 8 atomes de carbone, avec la condition qu'au moins un des symboles $R_4$ et $R_5$ représente l'hydrogène, |
| m | désigne un nombre entier de 1 à 40, et |
| n | désigne un nombre entier de 1 à 4, |

les motifs dérivant de la formule III étant liés par covalence, et en ce qu'on utilise, comme stabilisant de dispersion, un copolymère de l'anhydride maléique et d'un oxyde de vinyle et d'alkyle et/ou d'un ester vinylique et/ou d'une $\alpha$-oléfine à longue chaîne.

**2.** Procédé selon la revendication 1 caractérisé en ce qu'on obtient des polymères dans lesquels la quantité de (II) est d'au moins 95% en poids par rapport à la quantité totale de (I) et (II).

**3.** Procédé selon l'une des revendications 1 et 2, caractérisé en ce que la quantité des motifs correspondants à la formule III est comprise entre 1 et 20% en poids par rapport au polymère de base.

**4.** Procédé selon l'une quelconque des revendications 1 à 3, caractérisé en ce que $R_1$ et $R_2$, dans (I) ou (II), représentent chacun l'hydrogène.

**5.** Procédé selon l'une quelconque des revendications 1 à 4, caractérisé en ce que que $R_3$ représente un alkyle contenant de 4 à 30 atomes de carbone, X représente -O- et/ou -COO-, $R_4$ et $R_5$ représentent chacun l'hydrogène, m désigne un nombre de 5 à 35, et n est égal à 1 ou à 2.

**6.** Procédé selon l'une quelconque des revendications 1 à 5, caractérisé en ce que $R_3$ représente un alkyle contenant de 12 à 20 atomes de carbone, et X représente -O- et/ou -COO-.

**7.** Polymère selon la revendication 6 caractérisé en ce que X ne représente que -O-.

**8.** Procédé selon l'une quelconque des revendications 1 à 7, caractérisé en ce que le stabilisant de dispersion utilisé est un copolymère de l'anhydride maléique et d'un oxyde de vinyle et d'alkyle dont le radical alkyle contient de 6 à 30 atomes de carbone et/ou d'un ester vinylique dont le radical acyle contient de 6 à 30 atomes de carbone et/ou d'une $\alpha$-oléfine à longue chaîne qui contient de 8 à 30 atomes de carbone.

**9.** Procédé selon l'une quelconque des revendications 1 à 8, caractérisé en ce que le stabilisant de dispersion contient l'anhydride maléique et les comonomères dans un rapport d'environ 1 : 1.

**10.** Procédé selon l'une quelconque des revendications 1 à 9, caractérisé en ce qu'on utilise le stabilisant de dispersion en une quantité de 0,001 à 10% en poids par rapport à la quantité totale des monomères.

**11.** Procédé selon l'une quelconque des revendications 1 à 10, caractérisé en ce que l'oxyde de vinyle et d'alkyle est l'oxyde de vinyle et de stéaryle, et l'$\alpha$-oléfine à longue chaîne est l'octadécène-1.

**12.** Procédé selon l'une quelconque des revendications 1 à 11, caractérisé en ce qu'on utilise, comme composés de formule III, des alcools éthoxylés et/ou des acides carboxyliques éthoxylés, en des quantités de 1 à 30% en poids par rapport à la quantité totale des monomères.

**13.** Procédé selon la revendication 12 caractérisé en ce que les alcools sont des monoalcools primaires, les acides carboxyliques sont des monoacides carboxyliques primaires, et, dans les deux cas, le

radical hydrocarboné est un alkyle contenant de 4 à 30 atomes de carbone.

**14.** Procédé selon l'une quelconque des revendications 1 à 13, caractérisé en qu'on utilise, comme milieux de dispersion liquides, des hydrocarbures contenant de 6 à 20 atomes de carbone ou une huile de paraffine très fluide.

**15.** Application des polymères préparés selon l'une quelconque des revendications 1 à 14, éventuellement après réaction avec des "écarteurs", à la fabrication de substances biologiquement actives liées à des supports, comme agents d'adsorption ou pour la fabrication d'objets moulés.

**16.** Application selon la revendication 15 caractérisée en ce que les substances à activité biologique sont des enzymes.